# EUROPÄISCHE PATENTSCHRIFT

(11) **EP 0 946 469 B1**
(45) Veröffentlichungstag und Bekanntmachung des Hinweises auf die Patenterteilung: **27.03.2002**
(21) Anmeldenummer: 97954933.4
(22) Anmeldetag: 19.12.1997
(51) Int. Cl.: C07C 6/06

(54) **VON CYCLOPENTEN ABGELEITETE OLIGOMERENGEMISCHE, VERFAHREN ZU IHRER HERSTELLUNG UND IHRE VERWENDUNG**
OLIGOMER MIXTURES DERIVED FROM CYCLOPENTENE, METHOD FOR THE PRODUCTION AND USE THEREOF
MELANGES OLIGOMERES DERIVES DE CYCLOPENTENE, PROCEDES DE PREPARATION ET D'UTILISATION DESDITS MELANGES

(30) Priorität: 23.12.1996 DE 19654166
(43) Veröffentlichungstag der Anmeldung: 06.10.1999
(73) Patentinhaber: BASF AKTIENGESELLSCHAFT, 67056 Ludwigshafen (DE)
(72) Erfinder: SCHWAB, Peter, 67098 Bad Dürkheim (DE); SCHÄFER, Martin, Ludwigshafen (DE); HÖHN, Arthur, 67281 Kirchheim (DE)
(74) Vertreter: Kinzebach, Werner, Dr.
(86) Internationale Anmeldenummer: EP9707199
(87) Internationale Veröffentlichungsnummer: WO9828245

(56) Entgegenhaltungen:
- EP-A- 0 691 318
- EP-A- 0 742 234
- US-A- 3 527 828
- US-A- 3 622 644

## Beschreibung

Die vorliegende Erfindung betrifft von Cyclopenten abgeleitete Oligomerengemische, ein Verfahren zu ihrer Herstellung durch ringöffnende Metathese und ihre Verwendung als Zwischenprodukte für die Weiterverarbeitung durch Doppelbindungsfunktionalisierung.

Bei der Aufarbeitung von Erdöl durch Steamcracken fällt unter anderem ein als C₅-Schnitt bezeichnetes Kohlenwasserstoffgemisch mit einem hohen Gesamtolefinanteil von z.B. etwa 50% an, von denen ca. 15% auf Cyclopenten und der Rest auf acyclische Monoolefine, vor allem n-Penten (ca. 15 Gew.-%) und weitere isomere Pentene (ca. 20 Gew.-%) entfallen. Dieses Gemisch kann gewünschtenfalls vor der Weiterverarbeitung einer katalytischen Teilhydrierung unterzogen werden, so daß dann im wesentlichen keine Diene mehr enthalten sind. Zur Gewinnung des im C₅-Schnitt zu etwa 8% enthaltenen Cyclopentans, das beispielsweise als Ersatzstoff für die bezüglich der Schädigung der Atmosphäre bedenklichen FCKWs und HFCKWs als Treibmittel eingesetzt wird, sowie gegebenenfalls zur Gewinnung der übrigen gesättigten acyclischen Pentene, ist es nach dem Stand der Technik notwendig, den C₅-Schnitt einer destillativen Aufarbeitung zu unterziehen. Diese ist bei gleichzeitiger Anwesenheit von acyclischen und cyclischen C₅-Olefinen, insbesondere von Cyclopenten, verfahrenstechnisch sehr aufwendig. Daher besteht ein Bedarf an einem Verfahren zur nicht-destillativen Entfernung des Cyclopentens und gegebenenfalls weiterer Monoolefine aus dem C₅-Schnitt, möglichst unter gleichzeitiger Herstellung eines neuen Wertproduktes.

Eine technisch wichtige Reaktion der Olefine unter Erhalt der Anzahl an C=C-Doppelbindungen ist die Metathese. Der Begriff Metathese bezeichnet formal den Austausch der Alkylidengruppen zwischen zwei Alkenen in Gegenwart von homogenen oder heterogenen Übergangsmetallkatalysatoren. Ein einfaches Beispiel für eine großtechnisch genutzte Metathesereaktion zwischen zwei acyclischen Olefinen ist die Umwandlung von Propen in Ethen und 2-Buten.

Die nach dem Mechanismus der Metathese verlaufende ringöffnende Polymerisation von cyclischen Olefinen führt zu Poly-(1-alkenylenen), die als Polyalkenamere bezeichnet werden. Im Gegensatz zur Polymerisation von Vinylverbindungen bleiben dabei die Doppelbindungen des Monomers im Polymerisat erhalten. Die Metathese acyclischer und cyclischer Olefine wird z.B. von R.H. Grubbs in Progress in Inorganic Chemistry, John Wiley & Sons, New York, 1978, Bd. 24, S. 1-50; in Comprehensive Organomet. Chem., Pergamon Press, Ltd., New York, 1982, Bd. 8, S. 499-551 und in Science 1989, 243, S. 907-915; sowie von D.S. Breslow in Prog. Polym. Sci. 1993, Bd. 18, S. 1141-1195; von H. Höcker und H. Keul in Adv. Mater. 1994, 6, Nr. 1, S. 21-36 und von G. Sundararajan in J. Sci. Indus. Res. 1994, Bd. 53, S. 418-432 beschrieben.

R.R. Schrock beschreibt in Acc. Chem. Res. 1990, 23, S. 158 ff. den Mechanismus der ringöffnenden Metathesepolymerisation gemäß dem folgenden Schema:

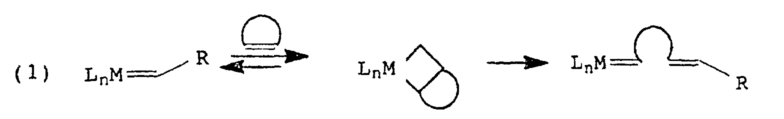

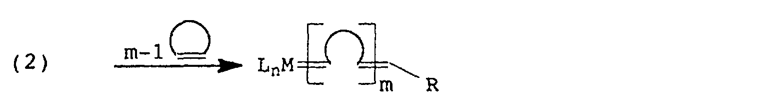

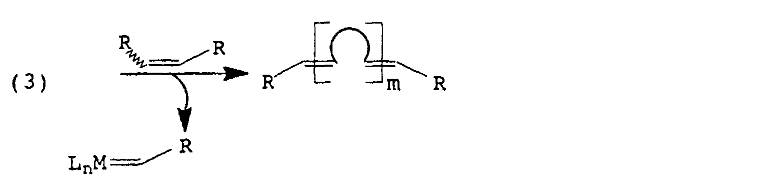

Dabei wird im Initiierungsschritt (1) zunächst ein Cycloolefin an einen Metallcarben-Komplex unter Ausbildung einer Metallacyclobutan-Zwischenstufe addiert, welche sich entweder in die Edukte zurückspalten oder zu einem neuen Carbenkomplex unter Kettenverlängerung öffnen kann. Das Kettenwachstum (Propagation) (2) erfolgt im Idealfall der lebenden ringöffnenden Metathesepolymerisation derart, daß pro Metallzentrum ein Polymerstrang gebildet wird, so daß monodisperse Polymere mit einer Uneinheitlichkeit von nahezu 1 erhalten werden. Der Kettenabbruch (3), d.h. die Ablösung des Polymers vom Metall, erfolgt im allgemeinen durch Zugabe eines acyclischen Olefins. Dabei ist im Idealfall sowohl eine gezielte Beeinflussung der Kettenlänge als auch eine Regeneration des Katalysators möglich. Bei Kettenabbruch mit Hilfe eines funktionalisierten Olefins überträgt sich die Funktionalität auf das Kettenende des Polymers.

Die BE-A-759774 beschreibt ein Verfahren zur Regelung der Molmasse und der Molmassenverteilung bei der ringöffnenden Metathesepolymerisation, wobei z.B. bei der Herstellung eines Polyoctenamers 1-Penten als acyclisches Olefin und somit Kettenabbruchreagens verwendet wird.

Durch den Einsatz acyclischer Olefine läßt sich zum einen die Kettenlänge bei der ringöffnenden Metathesepolymerisation gezielt steuern. Zum anderen dient die Ethenolyse cyclischer Olefine, bei der äquimolare Mengen eines cyclischen Olefins mit Ethen nach dem folgenden Schema: 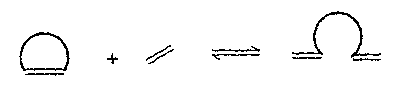 umgesetzt werden, zur Herstellung ansonsten schwer zugänglicher α,ω-ungesättigter Olefine.

Die US-A-3,715,410 beschreibt die Umsetzung cyclischer Olefine mit acyclischen Olefinen zu acyclischen Polyenen in Gegenwart eines sogenannten Olefindisproportionierungskatalysators. So lassen sich durch Umsetzung cyclischer Monoolefine oder nicht-konjugierter Polyolefine mit Ethen α,ω-ungesättigte acyclische Diolefine oder Polyene herstellen. α,ω-ungesättigte Diolefine können leicht zu Diolen mit terminalen Hydroxylgruppen umgesetzt werden und werden z.B. bei der Herstellung von Polyestern verwendet. Durch Umsetzung cyclischer Monoolefine mit einem substituierten acyclischen Olefin erhält man nicht-konjugierte acyclische Diolefine mit einer terminalen und einer endständig substituierten Doppelbindung, wie z.B. bei der Umsetzung von Cycloocten mit Propen zu 1,9-Undecadien. Diese werden z.B. als Monomere bei der Homo- oder Copolymerisation verwendet, um Polymere zu erhalten, die leicht vernetzbar sind.

Die DE-A-2 047 270 beschreibt ein Verfahren zur Herstellung eines Katalysators für die Disproportionierung von Olefinen auf Basis metallorganischer Verbindungen, die ein Übergangsmetall der 6. Nebengruppe und ein Metall der 1. bis 3. Hauptgruppe des Periodensystems, vorzugsweise Aluminium, umfassen. Diese Katalysatoren eignen sich sowohl für Homo- als auch Kreuzdisproportionierungen, bei denen ein Gemisch aus zwei verschiedenen Olefinen unter Bildung eines Produktgemisches nach dem folgenden allgemeinen Schema umgesetzt wird:

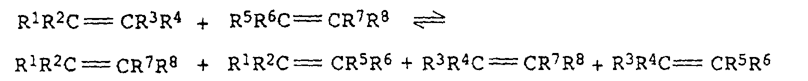

Zu den beschriebenen Disproportionierungsreaktionen zählen:
1. Die Umwandlung eines Gemisches aus einem acyclischen Mono- oder Polyolefin und einem cyclischen Mono- oder Polyolefin zu einem acyclischen Polyolefin von höherem Molekulargewicht.
2. Die Umwandlung eines oder mehrerer cyclischer Mono- oder Polyolefine unter Bildung cyclischer Polyolefine von höherem Molekulargewicht.
3. Die Umwandlung eines oder mehrerer acyclischer Polyolefine unter Bildung cyclischer Mono- oder Polyolefine und acyclischer Mono- oder Polyolefine.

Die DE-A-2 028 935 beschreibt Polyalkenamere, die durch ringöffnende Metathesepolymerisation aus cyclischen Olefinen und ungesättigten Estern mit nicht in einem Ring befindlichen Doppelbindungen hergestellt werden, wobei ein Katalysatorsystem bestehend aus einer Wolfram- oder Molybdänverbindung und einer aluminiumorganischen Verbindung sowie einer Verbindung, die eine oder mehrere Hydroxyl- und/oder Sulfhydryl-Gruppen enthält, verwendet wird.

Die DE-A-2 051 798 beschreibt ebenfalls ein Verfahren zur Herstellung von Polyalkenameren durch ringöffnende Polymerisation von cyclischen Olefinen mittels Katalysatoren bestehend aus:
a) Wolframhalogeniden oder Wolframoxihalogeniden mit 6-wertigem Wolfram,
b) einer aluminiumorganischen Verbindung,
c) einer im Reaktionsgemisch löslichen Monocarbonsäure.

Die DE-A-2 051 799 beschreibt auch ein Verfahren zur Herstellung von Polyalkenameren durch ringöffnende Polymerisation von cyclischen Olefinen mit einem Katalysatorsystem auf Basis einer 6-wertigen Wolframverbindung und einer aluminiumorganischen Verbindung.

Die DE-A-3 529 996 beschreibt ein Verfahren zur Herstellung von Polyalkenameren mit erhöhtem cis-Gehalt der im Polymeren vorhandenen Doppelbindungen, hergestellt durch ringöffnende Metathesepolymerisation von cis,cis-Cycloocta-1,5-dien oder Norbornen, wobei man die Polymerisation in Gegenwart eines offenkettigen 1,3-Diens, wie z.B. Isopren, oder eines cyclischen 1,3-Diens, wie z.B. Cyclohexa-1,3-dien durchführt.

Die DE-A-2 201 161 beschreibt ein Verfahren zur Herstellung von Polymerisaten aus Cycloolefinen, bevorzugt Cyclopenten und Cycloocten, durch ringöffnende Metathesepolymerisation mittels eines Katalysatorsystems, bestehend aus einem Übergangsmetallsalz, vorzugsweise einem (Oxi-)halogenid von Chrom, Molybdän oder Wolfram, einer metallorganischen Verbindung eines Elementes der 4. Hauptgruppe, bevorzugt einer Alkyl- oder Arylverbindung des Zinns oder Bleis, und einem Aluminiumhalogenid.

Die DE-A-2 343 093 beschreibt ein Verfahren zur ringöffnenden Polymerisation von cyclischen Olefinen an Katalysatoren, bestehend aus
a) einem Halogenid eines Metalls aus den Gruppen Vb oder VIb des Periodensystems,
b) einer metallorganischen Verbindung eines Metalls der Gruppen IIa, IIIa und IVa des Periodensystems,
c) gegebenenfalls einem Cokatalysator, der eine C=C-Doppelbindung in Konjugation zu einer C=O-Doppelbindung enthält, wie z.B. einer α,β-ungesättigten aliphatischen Mono- oder Polycarbonsäure. Dabei werden Polyalkenamere erhalten, die höhere Werte der Uneinheitlichkeit und damit eine verbreiterte Molekulargewichtsverteilung besitzen.

Die DE-A-2 424 298 beschreibt ein Verfahren zur Herstellung von Segmentpolymeren durch ringöffnende Metathesepolymerisation eines Cycloolefins mit einem hochmolekularen Oligoolefin, vorzugsweise einem α,ω-Diolefin mit zusätzlichen seitenständigen Doppelbindungen. Man erhält so Polymere mit maßgeschneiderten Produkteigenschaften.

Von den zuvor genannten Metatheseverfahren hat vor allem die Synthese konventionell schwer zugänglicher Olefine, Diolefine und speziell funktionalisierter Olefine eine breite großtechnische Anwendung gefunden. Das J. Mol. Catal. 15, S. 21 ff. (1982), beschreibt von der Phillips Petroleum Company entwickelte und industriell genutzte Verfahren der Olefinmetathese. Dabei wird unterschieden zwischen Verfahren zur Herstellung von Monoolefinen, zu denen z.B. der Triolefinprozeß zur Herstellung von hochreinem Ethylen und linearen Butenen aus Propen sowie der Neohexenprozeß zur Spaltung von Diisobutylen mit Ethylen zur Herstellung von Neohexen (3,3-Dimethyl-1-buten) und Isobutylen zählen, und Verfahren zur Herstellung von α,ω-ungesättigten Di- und Polyenen durch die zuvor schon beschriebene Ethenolyse cyclischer Olefine. Zur Herstellung funktionell substituierter Olefine bedarf es spezieller Metathese-Katalysatoren, die durch polare Substituenten nicht vergiftet werden. Diese basieren auf Heteroatomsubstituierten Carbenkomplexen, wie sie erstmals von E.O. Fischer hergestellt wurden, die durch Metall(oxi)halogenide aktiviert werden. Eine sehr aktive Cokatalysatorzusammensetzung erhält man z.B. durch Kombination von Zinn(IV)chlorid mit Silicium- oder Germaniumtetrachlorid.

R.L. Banks beschreibt in J. Mol. Catal. 8, S. 269 ff. (1980), ebenfalls industrielle Aspekte von Olefindisproportionierungsreaktionen.

Die US-A-3,652,703 beschreibt ein Verfahren zur Olefinmetathese unter Verwendung eines Katalysators auf Basis von Ruthenium auf einem Siliciumdioxidträger.

Die US-A-3,659,008 beschreibt ein Verfahren zur Herstellung nicht-konjugierter acyclischer Polyene mit 4 Kohlenstoffatomen zwischen den Doppelbindungen, umfassend die Disproportionierung von Ethylen und einem cyclischen Polyen mit 4 Kohlenstoffatomen zwischen den Doppelbindungen, wie z.B. 1,5-Cyclooctadien, wobei ein Gemisch aus gewünschten acyclischen Polyenen sowie schwereren und leichteren sekundären Polyenen erhalten wird.

Die US-A-3,660,507 beschreibt ein Verfahren zur Metathese von Olefinmischungen aus acyclischen und/oder cyclischen Olefinen an einem Katalysatorsystem, umfassen eine aktive Katalysatormasse und Magnesiumoxid.

Die US-A-3,792,102 beschreibt ebenfalls die Verwendung einer Magnesiumoxidschicht in einem Schichtbettkatalysator zur Disproportionierung von Mischungen aus acyclischen und cyclischen Olefinen zur Herstellung von Dienen.

Die US-A-3,865,751 beschreibt ebenfalls eine aktive Katalystorzusammensetzung für die Umwandlung von Olefinen, umfassend eine Mischung aus Magnesiumoxid und einem Olefindisproportionierungskatalysator.

Die US-A-3,836,480 beschreibt eine aktive Katalysatorzusammensetzung für die Umwandlung von Olefinen, umfassend einen Disproportionierungskatalysator und Magnesiumoxid, welches mit Kohlenmonoxid, Stickstoffmonoxid oder Wasserstoff behandelt wurde.

Die US-A-4,707,465 beschreibt einen verbesserten Olefindisproportionierungskatalysator, hergestellt durch Mischen eines hochschmelzenden anorganischen Oxids, welches eine katalytisch aktive Menge Molybdän- oder Wolframoxid enthält, mit einem Alkali- oder Erdalkalidithionitsalz als Promotor.

Die US-A-3,637,893 beschreibt ein Verfahren zur Disproportionierung zweier nicht-konjugierter Olefine an einem Disproportionierugnskatalysator, bestehend aus einem hochschmelzenden anorganischen Oxid, welches mindestens 0,1 Gew.-% Molybdän- oder Wolframoxid enthält, und einem perchlorierten Kohlenwasserstoff.

Die US-A-3,691,095 beschreibt ein Verfahren zur Herstellung von Katalysatoren für Olefindisproportionierungsreaktionen durch Umsetzung eines Übergangsmetallcarbonylkomplexes der allgemeinen Formel Aₙ(MM'(CO)_{q}Lₘ)⁻ⁿ, wobei A für ein Alkalimetallkation oder ein Ammonium-, Phosphonium- oder Arsonim-Kation steht, M für ein Metall der 6. Nebengruppe steht, M' für ein Metall der 7. oder 8. Nebengruppe steht, L für einen einzähnigen oder zweizähnigen Liganden, wie z.B. CO, NH₃, Hydrazin, etc. steht, mit einem Ammonium-, Phosphonium- oder Arsoniumhalogenid und einem metallorganischen Aktivator, der ein Organoaluminiumhalogenid umfaßt.

Das J. Am. Chem. Soc. 92, S. 528 ff. (1970), beschreibt homogene Katalysatoren für Olefindisproportionierungsreaktionen auf Basis von Nitrosyl-Molybdän und Wolframverbindungen. Die Katalysatoren werden durch Umsetzung von Nitrosyl-Molybdän und -Wolframderivaten mit Organoaluminiumverbindungen, wie z.B. C₂H₅AlCl₂ und (CH₃)₃Al₂Cl₃, erhalten und eignen sich für die meisten der vorgenannten Olefinmetathesereaktionen.

Eine weitere technische wichtige Metathesereaktion ist die Kreuzmetathese, die, wie zuvor bereits erwähnt, z.B. in der BE-A-759 774 beschrieben wird. Die C.R. Acad. Sc., Ser. C, S. 1077 ff. (1973) beschreibt die Metathese zwischen Cycloocta-1,5-dien und 4-Octen unter Verwendung eines WCl₆/EtAlCl₂/EtOH-Katalysatorsystems, wobei durch Kreuzmetathese zwischen jeweils einem acyclischen und einem cyclischen Mono- oder Oligomeren schließlich Polybuta-1,4-diene erhalten werden.

Die US-A-3,622,644 beschreibt ein Verfahren zur Herstellung von Oligomeren aus Ethen und einem cyclischen Monoolefin, bevorzugt Cyclopenten, unter Verwendung eines Katalysators, der
a) einen Rheniumkomplex der Formel (L)₂ReOX₃, worin X für Halogen und L für von dreiwertigem Phosphorarsen oder Antimon abgeleitete Liganden steht und
b) einer Organoaluminiumverbindung.

Die US-A-4,654,461 beschreibt ein Verfahren zur Herstellung von 1,5,9-Tetradecatrien mit hohem cis-Anteil durch Disproportionierung von 1,5-Cyclooctadien und 1-Hexen in Anwesenheit eines heterogenen Katalysators auf Basis von Molybdänoxid an einem Siliciumdioxidträger mit großer Oberfläche. Im Blickpunkt des Interesses dieser Schrift steht dabei ausschließlich das in einer Ausbeute von nur 9% erhaltene einfachste Kreuzmetatheseprodukt.

Die EP-B-0 235 742 beschreibt ein Verfahren zur Herstellung von 9-Alkenylestern, wobei in einem ersten Reaktionsschritt das Kreuzmetatheseprodukt von Cycloocten und einem α-Olefin mit 3 bis 12 Kohlenstoffatomen, ein 1,9-Alkadien, hergestellt wird. Dabei wird der Katalysator ausgewählt unter Siliciumdioxid, Aluminiumoxid, Aluminiumphosphat, Zirkoniumphosphat, Calciumphosphat, Magnesiumphosphat, Titanphosphat oder Thoriumoxid, welche einen Promotor enthalten.

Die Makromol. Chem. 141, S. 161 ff. (1970), beschreibt die Telomerisation von cyclischen Olefinen mit acyclischen Olefinen in Gegenwart von WOCl₄/Al(C₂H₅)₂Cl bzw. WOCl₄/Sn(C₄H₉)₄ als Katalysator. Als Telomerisation bezeichnet man eine Polymerisation, bei der ein Molekül AB, das sogenannte Telogen, mit n-Molekülen eines Monomeren M, dem sogenannten Taxogen, zu einem Oligomeren oder Polymeren A(M)ₙB, dem sogenannten Telomeren reagiert. An den Enden der durch Metathese erhaltenen Telomeren befinden sich zwei gleiche oder verschiedene Gruppen, die sich aus dem acyclischen Olefin durch eine nicht fortlaufende Reaktion gebildet haben. Die Kettenabbruchreaktion unter Bildung der Telomeren kann als Kreuzmetathese aufgefaßt werden. Die Verteilung der Telomeren gehorcht einem statistischen Gesetz und steht in Übereinstimmung mit dem folgenden Reaktionsschema, umfassend
a) die Disproportionierung acyclischer Olefine,
b) die Polymerisation cyclischer Olefine und
c) die Telomerisation cyclischer Olefine mit acyclischen Olefinen.

Dabei wird der postulierte Mechanismus an verschiedenen Telomerisationen, wie z.B. der von Cyclopenten und 2-Penten untersucht, wobei die experimentellen Produktverteilungen mit errechneten verglichen werden (unter Annahme des obigen Mechanismus). Aufgrund der guten Übereinstimmung erscheint der angenommene Mechanismus als realistisch. Bei der Telomerisation von Cyclopenten und 2-Penten wird mit den beiden verwendeten Katalysatorsystemen nur ein relativer Oligomerenanteil von etwa 30 Mol-% und weniger erhalten. Somit scheidet das beschriebene Verfahren für eine technische Herstellung von C₅-Oligomerengemischen aus. Ein Hinweis auf eine mögliche Verwendung von C₅-Olefingemischen aus Fraktionen der Erdölraffination als Eduktgemische findet sich nicht.

J. Lal, R.R. Smith und J.M. O'Connor beschreiben in Polym. Prepr. (Am. Chem. Soc., Div. Polym. Chem.) 13(2), S. 914 ff. (1972), ein Verfahren zur Herstellung von Polyenen durch Kreuzmetathese eines Cyclomonoolefins, wie z.B. Cycloocten, oder eines nicht-konjugierten Cyclodiens mit einem α-Olefin, wie z.B. 1-Hexen an einem WCl₆/EtAlCl₂/EtOH-Katalysator bei Umgebungstemperatur und Reaktionszeiten von etwa 1 bis 2 Stunden. Eine gaschromatographische Analyse der Kreuzmetatheseprodukte ergab ein Produktmaximum bei einer Kohlenstoffanzahl von 14. Die resultierenden Kreuzmetatheseprodukte wurden zur Erhöhung der Molmasse anschließend einer Copolymerisation mit dem zuvor verwendeten α-Olefin unterzogen.

In J. Org. Chem. 40, S. 775 ff. (1975) wird die Metathese von 1-Hexen und Cycloocten beschrieben. Man erhält drei verschiedene homologe Produktserien linearer nicht-konjugierter Polyene in Abhängigkeit von den primären Metatheseprodukten. Serie A sind Folgeprodukte von 1,9-Decadien der allgemeinen Formel 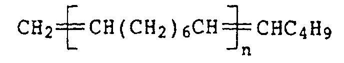 mit einer terminalen und einer innenliegenden endständigen Doppelbindung. Sie beruhen auf einer Kreuzmetathese zwischen Hexen und Cycloocten. Serie B sind Folgeprodukte von 5-Decen der allgemeinen Formel 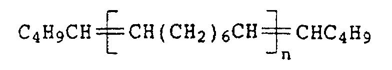 mit zwei innenliegenden endständigen Doppelbindungen. Sie beruhen auf einer Hexanselbstmetathese unter Bildung von 5-Decen und Ethen. Serie C sind Folgeprodukte von 1,9-Tetradecadien der allgemeinen Formel 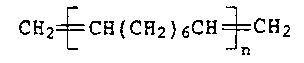 mit zwei terminalen endständigen Doppelbindungen. Sie beruhen auf einer Kreuzmetathese zwischen Cycloocten und dem in Serie B gebildeten Ethen. Das Hauptprodukt der Reaktion war C₁₄H₂₆ (1,9-Tetradecadien) aus der Kreuzmetathese von 1-Hexen und Cycloocten mit einem Gesamtanteil von 40 Gew.-%. Es konnten aber auch Reaktionsprodukte aus allen drei der zuvor genannten Serien mit höheren Molekulargewichten nachgewiesen werden, wobei der Gesamtcycloolefinumsatz größer als 70% war. Die verwendeten Katalysatoren waren von WCl₆ und einer organometallischen Verbindung abgeleitet, bevorzugt WCl₆/EtAlCl₂/EtOH und WCl₆/Bu₄Sn/2Et₂O.

Die US-A-3,527,828 beschreibt ein Verfahren zur Herstellung von Kohlenwasserstofftelomeren (Polyenen) durch Umsetzung eines acyclischen Monoolefins mit einem monocyclischen oder polycyclischen Olefin, umfassend bis zu 4 kondensierte Ringe und bis zu 4 nicht-konjugierte Doppelbindungen, an einem heterogenen Molybdän- oder Rheniumoxidkatalysator. Als Metathesebeispiele wurden die folgenden Eduktpaare aus cyclischem Olefin (Taxogen) und acyclischem Olefin (Telogen) umgesetzt, wobei die entsprechenden Polyene (Telomere) erhalten wurden:
a) Cyclopenten und Ethylen ergaben als Hauptprodukte 1,6,11-Dodecatrien (18,7% Selektivität) und 1,6,11,16-Heptdecatetraen (5,5% Selektivität) bei 35,6% Cyclopentenumsatz;
b) Cyclopenten und 1,6-Heptadien ergaben als Hauptprodukte 1,6,11-Dodecatrien (Selektivität 51%) und 1,6,11,17-Octadecatetraen (Selektivität 35%) bei einem Cyclopentenumsatz von 12%;
c) Cycloocten und Ethylen ergaben als Hauptprodukte 1,9-Decadien, 1,9,17-Octadecatrien und 1,9,17,25-Hexakosatetraen, wobei der Cyclooctenumsatz in Abhängigkeit von der Reaktionstemperatur zwischen 25 und 71% lag;
d) Norbornen und Ethylen ergaben überwiegend C₁₆-, C₂₃- und C₃₀-Telomere bei einer Umsetzungsrate des Norbonens von 62%.

Es findet sich in dieser und keiner der anderen zuvor genannten Schriften ein Hinweis auf eine mögliche Verwendung von C₅-Olefingemischen aus der Erdölraffination für die Herstellung von Oligomeren (Telomeren).

Die EP-A-691 318 beschreibt ein Verfahren zur Herstellung eines C₄-Olefingemisches, welches vor allem Isobuten und 1-Buten sowie Propen enthält. Dabei wird ein z.B. beim Steamcracken von Naphtha anfallendes olefinhaltiges C₅-Kohlenwasserstoffgemisch zuerst einer Hydrierung mit anschließender Fraktionierung unterzogen und dann in einer Metathesereaktion mit Ethen umgesetzt. Als Metathesekatalysatoren werden dabei ausschließlich heterogene Re-, W-, Mo- und Co-Oxidkatalysatoren verwendet. Die der Schrift zugrundeliegende Aufgabe ist die Bereitstellung eines Verfahrens zur Herstellung isomerer Butene, also von Olefinen mit einem geringeren Molekulargewicht als die im Ausgangskohlenwasserstoffgemisch (C₅-Schnitt) enthaltenen. Zur Lösung dieser Aufgabe erfolgt die Metathesereaktion in Gegenwart eines bis zu 10-fachen molaren Überschusses von Ethen, wobei die bei diesem Verfahren unerwünschte Selbstmetathese des C₅-Schnittes unterdrückt wird. Auf die Möglichkeit zur Nutzung der Selbstmetathese für die Oligomerisierung der im C₅-Schnitt enthaltenen cyclischen und acyclischen Olefine im Sinne einer ringöffnenden Metathesepolymerisation findet sich in der Schrift kein Hinweis.

Die EP-A-0 742 234 beschreibt ein Verfahren zur Umwandlung eines C4-Schnittes in Polyisobuten und Propen, wobei der C₄-Schnitt: 1.) einer Selektivhydrierung von Diolefinen mit gleichzeitiger Isomerisierung von 1-Buten in 2-Buten, 2.) einer Polymerisation des Isobutens zu Polyisobuten und 3.) einer Metathese des zurückbleibenden C₄-Schnittes mit Ethen zur Gewinnung von Propen, unterzogen wird.

Der vorliegenden Erfindung liegt die Aufgabe zugrunde, ein Verfahren zur Aufarbeitung eines C₅-Gemisches aus der Erdölverarbeitung (C₅-Schnitt), welches neben gesättigen Kohlenwasserstoffen noch cyclische und acyclische Monoolefine enthält, zur Verfügung zu stellen, wobei die Olefine möglichst unter Umwandlung in ein Wertprodukt von den acyclischen Kohlenwasserstoffen abgetrennt werden sollen. Vorteilhafterweise weist dazu das Wertprodukt ein höheres Molekulargewicht als die Olefine auf.

Überraschenderweise wurde nun gefunden, daß die Aufgabe durch ein Verfahren zur Herstellung von von Cyclopenten abgeleiteten Oligomerengemischen gelöst wird, wobei man den C₅-Schnitt einer Metathesereaktion unterwirft. Die dabei erfindungsgemäß hergestellten neuen Oligomeren eignen sich z.B. als Zwischenprodukte für durch Funktionalisierung der Doppelbindungen im Sinne einer polymeranalogen Umsetzung erhältliche Endprodukte.

Gegenstand der Erfindung ist somit ein Verfahren zur Herstellung von Oligomerengemischen der Formel I 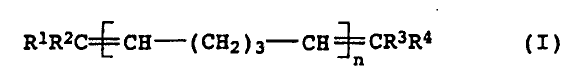 die von Cyclopenten abgeleitet sind,
worin
n für eine ganze Zahl von 1 bis 15 steht,
R¹, R², R³ und R⁴ unabhängig voneinander für Wasserstoff oder Alkyl stehen,
das dadurch gekennzeichnet ist, daß man ein Cyclopenten sowie acyclische Monoolefine enthaltendes, aus der Erdölverarbeitung durch Cracken erhaltenes Kohlenwasserstoffgemisch (C₅-Schnitt) mit einem Cyclopentengehalt von mindestens. 5 Gew.-% und einem Anteil von Pentenisomeren an den acyclischen Monoolefinen von mindestens 70 Gew.-% in einer homogen oder heterogen katalysierten Metathesereaktion umsetzt.

Der Wert n in der Formel I steht für die Anzahl der in die von Cyclopenten abgeleiteten Oligomere durch ringöffnende Metathesereaktion eingeführten Cyclopenten-Einheiten. Vorzugsweise erhält man nach dem erfindungsgemäßen Verfahren Oligomerengemische, bei denen ein möglichst großer Anteil, wie z.B. mindestens 40 Gew.-% (ermittelt durch die Flächenintegration der Gaschromatogramme; Gerät: Hewlett Packard; Detektor: Flammenionisations-Detektor; Säule: DB 5,30 m x 0,32 mm, Belegung 1 µ; Temperaturprogramm: 60°C 5 min, isotherm, Heizrate 10°C/min Max: 300°C) einen Wert von n > 1 aufweist. Der Wert n und somit der Grad der ringöffnenden Metathese kann, wie weiter unten ausgeführt, durch die Aktivität des verwendeten Metathesekatalysators beeinflußt werden.

Die Reste R¹, R², R³ und R⁴ stehen unabhängig voneinander für Wasserstoff oder Alkyl, wobei der Ausdruck "Alkyl" geradkettige und verzweigte Alkylgruppen umfasst.

Vorzugsweise handelt es sich dabei um geradkettige oder verzweigte C₁-C₁₅-Alkyl-, bevorzugt C₁-C₁₀-Alkyl-, insbesondere bevorzugt C₁-C₅-Alkylgruppen. Beispiele für Alkylgruppen sind insbesondere Methyl, Ethyl, Propyl, 1-Methylethyl, Butyl, 1-Methylpropyl, 2-Methylpropyl 1,1-Dimethylethyl, n-Pentyl, 1-Methylbutyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Di-methylbutyl, 3,3-Dimethylbutyl, 1,1,2-Trimethylpropyl, 1,2,2-Trimethylpropyl, 1-Ethylbutyl, 2-Ethylbutyl, 1-Ethyl-2-methylpropyl, n-Heptyl, 1-Methylhexyl, 1-Ethylpentyl, 2-Ethylpentyl, 1-Propylbutyl, Octyl, Decyl, Dodecyl, etc.

Der Verzweigungsgrad und die Anzahl der Kohlenstoffatome der endständigen Alkylreste R¹, R², R³ und R⁴ hängen von der Struktur der acyclischen Monoolefine des verwendeten Kohlenwasserstoffgemisches und der Aktivität des Katalysators ab. Wie im folgenden genauer beschrieben wird, beeinflußt die Aktivität des Katalysators den Grad der Kreuzmetathese (Selbstmetathese) der acyclischen Olefine unter Bildung strukturell neuer Olefine, in die dann formal Cyclopenten im Sinne einer ringöffnenden Metathesepolymerisation insertiert wird.

Vorzugsweise werden durch das erfindungsgemäße Verfahren Oligomerengemische hergestellt, die einen erhöhten Anteil an Oligomeren mit nur einer terminalen Doppelbindung aufweisen.

Die Metathesereaktion umfaßt formal
a) die Disproportionierung der acyclischen Monoolefine des Kohlenwasserstoffgemisches durch Kreuzmetathese,
b) die Oligomerisation des Cyclopentens durch ringöffnende Metathese,
c) den Kettenabbruch durch Umsetzung der Oligomere aus b) mit einem acyclischen Olefin des Kohlenwasserstoffgemisches oder eines Produktes aus a),
wobei die Schritte a) und/oder b) und/oder c) mehrmals für sich alleine oder in Kombination durchlaufen werden können.

### Schritt a)

Die Kreuzmetathese der acyclischen Monoolefine soll am Beispiel der Metathese von 1-Penten und 2-Penten erläutert werden:

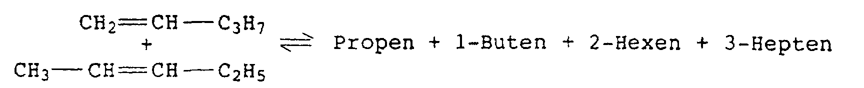

Durch Kombinationen aus Kreuzmetathese verschiedener und Selbstmetathese gleicher acyclischer Olefine, wie z.B. der Selbstmetathese von 1-Penten zu Ethen und 4-Octen sowie durch mehrmaliges Durchlaufen dieser Reaktion werden eine Vielzahl von Monoolefinen mit unterschiedlicher Struktur und Kohlenstoffatomanzahl erhalten, die die Endgruppen der erfindungsgemäßen Oligomeren bilden. Über den Anteil an Kreuzmetatheseprodukten, der mit steigender Aktivität des verwendeten Katalysators zunimmt, wird auch der Doppelbindungsanteil der Oligomeren beeinflußt. So wird z.B. bei der zuvor beschriebenen Selbstmetathese von 1-Penten Ethen freigesetzt, welches gegebenenfalls gasförmig entweichen kann, wobei ein Doppelbindungsäquivalent der Umsetzung entzogen wird. Gleichzeitig steigt der Anteil an Oligomeren ohne terminale Doppelbindungen. So wird in dem obigen Beispiel z.B. durch Insertion von Cyclopenten in 4-Octen ein Oligomer ohne terminale Doppelbindungen gebildet.

### Schritt b)

Die durchschnittliche Anzahl an Cyclopenten-Insertionen in die wachsende Kette im Sinne einer ringöffnenden Metathesepolymerisation bestimmt das mittlere Molekulargewicht des gebildeten Cyclopenten-Oligomerengemisches. Vorzugsweise werden durch das erfindungsgemäße Verfahren Oligomerengemische mit einem mittleren Molekulargewicht von mindestens 274 g pro Mol gebildet, was einer durchschnittlichen Anzahl von drei Cyclopenteneinheiten pro Oligomer entspricht.

### Schritt c)

Der Kettenabbruch erfolgt durch Umsetzung eines Oligomeren, welches noch ein aktives Kettenende in Form eines Katalysatorkomplexes (Alkylidenkomplexes) aufweist, mit einem acyclischen Olefin, wobei im Idealfall ein aktiver Katalysatorkomplex zurückgewonnen wird. Dabei kann das acyclische Olefin unverändert aus dem ursprünglich zur Reaktion eingesetzten Kohlenwasserstoffgemisch stammen oder zuvor in einer Kreuzmetathese nach Stufe a) modifiziert worden sein.

Das erfindungsgemäße Verfahren eignet sich ganz allgemein zur Herstellung von Oligomeren aus Kohlenwasserstoffgemischen, die acyclische und cyclische Olefine enthalten.

Vorzugsweise wird ein bei der Erdölverarbeitung großtechnisch anfallendes Kohlenwasserstoffgemisch verwendet, das gewünschtenfalls zur Entfernung von Dienen zuvor einer katalytischen Teilhydrierung unterzogen werden kann. Besonders geeignet für die Verwendung im vorliegenden Verfahren ist z.B. ein an gesättigten und ungesättigten C₅-Kohlenwasserstoffen angereichertes Gemisch (C₅-Schnitt). Zur Gewinnung des C₅-Schnittes kann z.B. beim Steamcracken von Naphtha anfallendes Pyrolysebenzin zuerst einer Selektivhydrierung unterzogen werden, um die enthaltenen Diene und Acetylene selektiv in die entsprechenden Alkane und Alkene zu überführen und anschließend einer fraktionierten Destillation unterworfen werden, wobei zum einen der für weitere chemische Synthesen wichtige C₆-C₈-Schnitt, der die aromatischen Kohlenwasserstoffe enthält, als auch der für das erfindungsgemäße Verfahren verwendete C₅-Schnitt anfallen.

Der C₅-Schnitt weist im allgemeinen einen Gesamtolefingehalt von mindestens 30 Gew.-%, bevorzugt mindestens 40 Gew.-%, insbesondere mindestens 50 Gew.-% auf.

Geeignet sind dabei C₅-Kohlenwasserstoffgemische mit einem Cyclopentengesamtgehalt von mindestens 5 Gew.-%, bevorzugt mindestens 10 Gew.-%, insbesondere mindestens 12 Gew.-%, und im allgemeinen nicht mehr als 30 Gew.-%, vorzugsweise nicht mehr als 20 Gew.-%.

Des weiteren weisen geeignete C₅-Kohlenwasserstoffgemische einen Anteil von Pentenisomeren an den acyclischen Monoolefinen von mindestens 70 Gew.-%, bevorzugt mindestens 80 Gew.-%, insbesondere mindestens 90 Gew.-% auf.

Nach einer bevorzugten Ausführungsform des erfindungsgemäßen Verfahrens wird ein großtechnisch anfallender C₅-Schnitt mit einem Gesamtolefinanteil von z.B. 50 bis 60 Gew.-%, wie etwa 56%, einem Cyclopentenanteil von z.B. 10 bis 20 Gew.-%, wie etwa 15 Gew.-% und einem Anteil an Pentenisomeren von z.B. 33 bis 43 Gew.-%, wie etwa 38 Gew.-%, wobei etwa 16 Gew.-% auf das n-Penten und etwa 22 Gew.-% auf isomere Pentene entfallen, verwendet.

Nach einer speziellen Ausführungsform des erfindungsgemäßen Verfahrens wird ein Kohlenwasserstoffgemisch verwendet, welches den C₅-Schnitt und eine acyclische C₄-Olefine enthaltende Erdölfraktion (Raffinat-2) umfaßt.

Nach einer weiteren speziellen Ausführungsform des erfindungsgemäßen Verfahrens wird ein Kohlenwasserstoffgemisch verwendet, welches den C₅-Schnitt und Ethen umfaßt. Dabei werden Oligomerengemische mit einem erhöhten Doppelbindungsanteil erhalten. Dies wird zum einen durch Ethenolyse der im C₅-Schnitt enthaltenen acyclischen n- und iso-Pentene zu kürzerkettigen α-Olefinen, wie Propen und 1-Buten, erreicht, die mit Cyclopenten in einer ringöffnenden Metathesereaktion unter Bildung von Oligomeren mit jeweils einer terminalen Doppelbindung reagieren. In Gegenwart von Ethen wird auch die Selbstmetathese der acyclischen Olefine unter Bildung weiteren Ethens, wie z.B. die Selbstmetathese von 1-Penten zu Ethen und 4-Octen, welches als Kettenabbruchreagenz zu Produkten ohne terminale Doppelbindungen führt, unterdrückt. Zum anderen wird eine weitere Erhöhung des Doppelbindungsanteils durch die Ethenolyse von Cyclopenten mit Ethen zu 1,6-Heptadien erzielt. Dabei entstehen Oligomerenfolgen, die jeweils über zwei terminale Doppelbindungen verfügen. Vorzugsweise resultieren bei einer Verwendung der so erhaltenen Oligomerengemische mit erhöhtem Doppelbindungsanteil zur Funktionalisierung Oligomerengemische mit erhöhter Funktionalitätsdichte.

Bei Verwendung eines Kohlenwasserstoffgemischs, welches den C₅-Schnitt und Ethen umfasst, wird die Reaktion bevorzugt bei einem Druck von 1 bis 200 bar, bevorzugter 20 bis 150 bar und insbesondere 40 bis 100 bar durchgeführt.

Geeignete Katalysatoren für die Metathese sind nach dem Stand der Technik bekannt und umfassen homogene und heterogene Katalysatorsysteme. Dabei wird ausdrücklich auf die zuvor als Stand der Technik beschriebenen Katalysatorsysteme Bezug genommen. Im allgemeinen basieren die für das erfindungsgemäße Verfahren geeigneten Katalysatoren auf einem Übergangsmetall der 6., 7. oder 8. Nebengruppe des Periodensystems, wobei vorzugsweise Katalysatoren auf Basis von Mo, W, Re und Ru verwendet werden.

Geeignete homogene Ratalysatorsysteme sind im allgemeinen Übergangsmetallverbindungen, die gegebenenfalls in Kombination mit einem Cokatalysator und/oder gegebenenfalls in Gegenwart der Olefinedukte befähigt sind, einen katalytisch aktiven Metallcarben-komplex zu bilden. Solche Systeme werden z.B. von R.H. Grubbs in Comprehensive Organomet. Chem., Pergamon Press, Ltd., New York, Bd. 8, S. 499 ff. (1982) beschrieben.

Geeignete Katalysator/Cokatalysator-Systeme auf W-, Mo- und Re-Basis können z.B. mindestens eine lösliche Übergangsmetallverbindung und ein alkylierendes Agens umfassen. Dazu zählen z.B. MoCl₂(NO)₂(PR₃)₂/Al₂(CH₃)₃Cl₃; WCl₆/BuLi;
WCl₆/EtAlCl₂(Sn(CH₃)₄)/EtOH; WOCl₄/Sn(CH₃)₄;
WOCl₂(O-[2,6-Br₂-C₆H₃])/Sn(CH₃)₄; CH₃ReO₃/C₂H₅AlCl₂, wobei die vier letztgenannten für das erfindungsgemäße Verfahren bevorzugt sind.

Weitere als Metathese-Katalysatoren geeignete Übergangsmetall-Alkyliden-Komplexe werden von R.R. Schrock in Acc. Chem. Res., 23, S. 158 ff. (1990) beschrieben. Allgemein handelt es sich um vierfach koordinierte Mo- und W-Alkylidenkomplexe, die zusätzlich zwei sperrige Alkoxy- und einen Imido-Liganden aufweisen. Dabei werden für das erfindungsgemäße Verfahren bevorzugt ((CH₃)₃CO)₂Mo(=N-[2,6-(i-C₃H₇)₂-C₆H₃])(=CHC(CH₃)₂C₆H₅) und [(CF₃)₂C(CH₃)O]₂Mo(=N-[2,5-(i-C₃H₇)-C₆H₃])(=CH(CH₃)₂C₆H₅) verwendet.

Besonders bevorzugt werden als homogene Metathesekatalysatoren die Katalysatoren verwendet, die in der Angew. Chem. 107, S. 2179 ff. (1995), in J. Am. Chem. Soc. 118, S. 100 ff. (1996) sowie in J. Chem. Soc., Chem. Commun., S. 1127 ff. (1995) beschrieben sind. Dazu zählen insbesondere RuCl₂(=CHR)(PR'₃)₂, bevorzugt RuCl₂(=CHC₆H₅)(P(C₆H₁₁)₃)₂, (η⁶-p-Cymol)RuCl₂(P(C₆H₁₁)₃)/(CH₃)₃SiCHN₂ und (η⁶-p-Cymol)RuCl₂(P(C₆H₁₁)₃)/C₆H₅CHN₂. Dabei werden die letzteren beiden aus einem Moläquivalent (η⁶-p-Cymol)RuCl₂(P(C₆H₁₁)₃) und 3 Moläquivalenten Diazoalkan ((CH₃)₃SiCHN₂ oder C₆H₅CHN₂) "in situ" erzeugt.

Geeignete heterogene Katalysatorsysteme umfassen im allgemeinen eine Übergangsmetallverbindung auf einem inerten Träger, die befähigt ist ohne Cokatalysator, durch Reaktion mit den Olefinedukten einen katalytischen aktiven Alkylidenkomplex zu bilden. Im erfindungsgemäßen Verfahren werden bevorzugt Re₂O₇ und CH₃ReO₃ verwendet.

Geeignete anorganische Träger sind die hierfür üblichen Oxide, insbesondere Silicium- und Aluminiumoxide, Alumosilikate, Zeolithe, Carbide, Nitride, etc. und deren Mischungen. Bevorzugt werden als Träger Al₂O₃, SiO₂ und deren Mischungen, gegebenenfalls in Kombination mit B₂O₃ und Fe₂O₃ verwendet.

Die zuvor genannten homogenen und heterogenen Katalysatorsysteme unterscheiden sich stark in ihrer katalytischen Aktivität, so daß die einzelnen Katalysatoren unterschiedliche optimale Reaktionsbedingungen für die Metathese aufweisen. Wie zuvor bereits beschrieben, beeinflußt die katalytische Aktivität bezüglich der Kreuzmetathese (Schritt a)) auch die Produktverteilung der von Cyclopenten abgeleiteten Oligomerengemische der Formel I. So sind die auf Ruthenium-basierenden homogenen Katalysatorsysteme RuCl₂(=CHC₆H₅)(P(C₆H₁₁)₃)₂, (η⁶-p-Cymol)RuCl₂(P(C₆H₁₁)₃)/(CH₃)₃SiCHN₂ und (η⁶-p-Cymol)RuCl₂(P(C₆H₁₁)₃)/C₆H₅CHN₂ für das erfindungsgemäße Verfahren besonders geeignet. Dabei weist der erstgenannte Rutheniumkomplex eine höhere katalytische Aktivität als die beiden letztgenannten auf, was bei ansonsten gleichen Reaktionsbedingungen zu höheren Raum-Zeit-Ausbeuten führt. Gleichzeitig tritt beim ersten Komplex jedoch auch vermehrt die Kreuzmetathese auf, wobei zum Teil auch Ethen freigesetzt wird und somit das erhaltene von Cyclopenten abgeleitete Oligomerengemisch einen etwas geringeren Anteil an Doppelbindungen aufweist, was sich z.B. in einer geringeren Jodzahl ausdrückt. Zudem steht aufgrund der Kreuzmetathese eine größere Anzahl an acyclischen Olefinen ohne endständige Doppelbindungen zur Verfügung, so daß mit dem erstgenannten homogenen Rutheniumkatalysator vermehrt von Cyclopenten abgeleitete Oligomere der Formel I erhalten werden, die nur eine oder keine terminale Doppelbindung aufweisen. Die beiden letztgenannten Rutheniumkomplexe weisen eine etwas geringere katalytische Aktivität als der erstgenannte auf, so daß mit ihnen nach dem erfindungsgemäßen Verfahren von Cyclopenten abgeleitete Oligomerengemische der Formel I erhalten werden, die einen höheren Doppelbindungsanteil und somit eine höhere Jodzahl sowie einen größeren Anteil an terminalen Doppelbindungen aufweisen.

Auch die heterogenen Katalysatorsysteme weisen die zuvor beschriebenen Aktivitätsunterschiede mit der entsprechenden Beeinflussung der Metatheseprodukte auf. Wird für das erfindungsgemäße Verfahren CH₃ReO₃ auf Al₂O₃ als heterogener Katalysator verwendet, so weist dieser eine höhere katalytische Aktivität als das entsprechende homogene Katalysatorsystem aus CH₃ReO₃/(C₂H₅)AlCl₂ auf.

Vorteilhafterweise wird als heterogener Katalysator Re₂O₇ auf Al₂O₃ verwendet. Dieser weist eine dem RuCl₂(=CHC₆H₅)(P(C₆H₁₁)₃)₂ annähernd vergleichbare Aktivität sowie eine ähnliche Produktverteilung auf und kann nach Regeneration im Luftstrom bei erhöhten Temperaturen, wie z.B. etwa 550°C erneut eingesetzt werden.

Gewünschtenfalls können somit je nach verwendetem Katalysator von Cyclopenten abgeleitete Oligomerengemische mit wechselnden Doppelbindungsanteilen und wechselnden Anteilen an terminalen Doppelbindungen erhalten werden.

Nach einer besonders bevorzugten Ausführungsform des erfindungsgemäßen Verfahrens wird als Metathesekatalysator ein homogener Katalysator auf Rutheniumbasis, ausgewählt unter RuCl₂(=CHC₆H₅)(P(C₆H₁₁)₃)₂, (η⁶-p-Cymol)RuCl₂(P(C₆H₁₁)₃)/(CH₃)₃SiCHN₂ und (η⁶-p-Cymol)RuCl₂(P(C₆H₁₁)₃}/C₆H₅CHN₂, verwendet, der dem Reaktionsgemisch als Lösung in einem organischen Lösungsmittel zugegeben wird. Geeignete Lösungsmittel sind z.B. aromatische Kohlenwasserstoffe, wie Toluol und Xylol sowie halogenierte Alkane, wie CH₂Cl₂, CHCl₃, etc.

Die Reaktionstemperatur liegt bei reaktiven Katalysatorsystemen bei -20 bis 200°C, bevorzugt 0 bis 100°C, insbesondere 20 bis 80°C.

Die Reaktion kann bei einem erhöhten Druck von bis zu 5 bar, bevorzugt bis zu 2 bar, oder insbesondere bevorzugt bei Umgebungsdruck durchgeführt werden.

Nach einer weiteren besonders bevorzugten Ausführungsform des erfindungsgemäßen Verfahrens wird als Metathesekatalysator ein heterogener Katalysator auf Rhenium-Basis, ausgewählt unter CH₃ReO₃/Al₂O₃ und bevorzugt Re₂O₇/Al₂O₃ verwendet, der dem Reaktionsgemisch ohne Lösungsmittelzusatz zugegeben wird.

Die Reaktionstemperatur liegt bei diesen, im Vergleich zu den vorgenannten homogenen Katalysatorsystemen etwas weniger aktiven Katalysatoren bei etwa 20 bis 120°C, bevorzugt 30 bis 100°C, insbesondere 40 bis 80°C.

Die Reaktion wird vorzugsweise bei einem erhöhten Druck von 2 bis 20 bar, bevorzugt 3 bis 15 bar, insbesondere 4 bis 12 bar durchgeführt.

Die verfahrenstechnische Ausführung des erfindungsgemäßen Verfahrens kann sowohl kontinuierlich als auch diskontinuierlich erfolgen. Geeignete Reaktionsapparaturen sind dem Fachmann bekannt und werden z.B. in Ullmanns Enzyklopädie der technischen Chemie, Bd. 1, S. 743 ff. (1951) beschrieben. Dazu zählen für das diskontinuierliche Verfahren z.B. Rührkessel und für das kontinuierliche Verfahren z.B. Rohrreaktoren.

Nach einer geeigneten diskontinuierlichen Variante des erfindungsgemäßen Verfahrens kann z.B. der C₅-Schnitt an einem der zuvor als bevorzugt beschriebenen, homogenen Rutheniumkatalysatoren, welcher gewünschtenfalls im Reaktorbehälter "in situ" erzeugt wird, in einer Metathesereaktion zu von Cyclopenten abgeleiteten Oligomerengemischen der Formel I umgesetzt werden.

Nach einer weiteren geeigneten kontinuierlichen Variante des erfindungsgemäßen Verfahrens kann z.B. der C₅-Schnitt an einem der zuvor als bevorzugt beschriebenen heterogenen Rheniumkatalysatoren in einem Rohrreaktor umgesetzt werden.

Nach beiden möglichen Verfahrensvarianten werden in Abhängigkeit vom verwendeten Katalysator und den übrigen Reaktionsparametern, vor allem der Reaktionstemperatur, Raum-Zeit-Ausbeuten von mindestens 10 g l⁻¹ h⁻¹, bevorzugt mindestens 15 g l⁻¹ h⁻¹, erzielt. Je nach Aktivität des Katalysators können jedoch auch wesentlich höhere Raum-Zeit-Ausbeuten bis etwa 500 g l⁻¹ h⁻¹ erzielt werden.

Die Auftrennung des Reaktionsgemisches erfolgt nach üblichen Verfahren. Dazu zählt z.B. die fraktionierte Destillation, gegebenenfalls unter vermindertem Druck, oder die Trennung bei erhöhten Temperaturen und Normaldruck in einem Fallfilmverdampfer. Dabei können leichtsiedende Fraktionen, die noch nicht umgesetzte Olefine enthalten, gewünschtenfalls in die Reaktionsapparatur zurückgeführt werden. Vorteilhafterweise wird bei dem erfindungsgemäßen Verfahren eine weitgehende Umsetzung der im C₅-Schnitt enthaltenen Olefine zu Oligomeren erzielt, so daß die abgetrennten Leichtsieder ein C₅-Kohlenwasserstoffgemisch mit überwiegend gesättigten cyclischen und acyclischen Verbindungen umfassen. Diese können, gegebenenfalls nach weiterer destillativer Auftrennung in Cyclopentan und n-/iso-Pentan-Gemische einer großtechnischen Verwendung zugeführt werden. Cyclopentan wird beispielsweise als Ersatzstoff für die bezüglich der Schädigung der Atmosphäre bedenklichen FCKWs und HFCKWs als Treibmittel für Polyurethan-Systeme zur Herstellung von Hartschäumen eingesetzt. n-/iso-Pentan-Gemische dienen z.B. als Lösungsmittel zum Schäumen von Polymeren sowie als Treibmittel für Aerosole.

Ein weiterer Gegenstand der Erfindung sind die nach dem erfindungsgemäßen Verfahren erhaltenen von Cyclopenten abgeleiteten Oligomerengemische der Formel I. Wie zuvor beschrieben, kann die Anzahl und die Lage der Doppelbindungen in den Oligomeren durch die Reaktionsbedingungen, insbesondere den jeweils verwendeten Katalysator beeinflußt werden. Nach dem erfindungsgemäßen Verfahren werden Cyclopenten-Oligomere erhalten, wobei die Jodzahl mindestens 250 g I₂/100 g Oligomere, bevorzugt mindestens 300 g I₂/100 g Oligomere beträgt.

Das mittlere Molekulargewicht der von Cyclopenten abgeleiteten Oligomere beträgt mindestens 274 g/mol, was einem mittleren Umsatz von drei Cyclopenten-Einheiten pro Oligomer entspricht, wobei in diesem Fall ein Kettenabbruch durch ein acyclisches Penten (und nicht durch ein Kreuzmetatheseprodukt) angenommen wird.

Besonders bevorzugt dienen die Oligomerengemische der Formel I als Zwischenprodukte für die Weiterverarbeitung durch Funktionalisierung wenigstens eines Teils der in ihnen enthaltenen Doppelbindungen. Diese kann im Sinne einer polymeranalogen Umsetzung, wie z.B. durch katalytische Hydroformylierung, erfolgen.

Die so erhaltenen Oxoprodukte weisen eine Vielzahl von Anwendungsmöglichkeiten, z.B. als Zusätze in Dichtmassen, Verträglichkeitsvermittlern, Klebstoffen, Inkrustationsinhibitoren, Polyelektrolyten, Komplexbildnern, Gerbstoffadditiven, etc. auf.

Die Erfindung wird anhand der folgenden nicht einschränkenden Beispiele erläutert.

### Beispiele

Für die Aufnahme der Gaschromatogramme wurde ein 5890 Gaschromatograph der Fa. Hewlett Packard mit einer DB 5,30 m x 0,32 mm Glaskapillarsäure und einem Flammionisationsdetektor mit angeschlossener Integriereinheit verwendet.

### Beispiel 1: (Modellsystem)

Ein 1:1-Gemisch aus je 17,1 mol Cyclopenten und 1-Penten wurde bei Raumtemperatur und Normaldruck mit einer "in situ"-erzeugten Katalysatormischung aus 8,6 mmol (p-Cymol)RuCl₂(PCy₃) und 2 ml Me₃SiCHN₂ in 50 ml CH₂Cl₂ versetzt. Hierbei wurde eine schwache Gasentwicklung beobachtet. Nach 3 Stunden Rühren wurde die Lösung über neutrales Al₂O₃ chromatographiert und das farblose Filtrat destillativ von nicht umgesetzten Leichtsiedern befreit. Es verblieben 956 g einer farblosen, niederviskosen Flüssigkeit nachfolgender Zusammensetzung (GC-Flächenprozent):
26% C₁₀H₁₈, 22% C₁₅H₂₆, 17% C₂₀H₃₄, 13% C₂₅H₄₂, 10% C₃₀H₅₀, 7% C₃₅H₅₈, 5% C₄₀H₆₆.
Jodzahl: 351 g J₂/100 g

### Beispiel 2: (homogen katalysierte Metathese von C₅-Schnitt)

1 1 C₅-Schnitt (Cyclopenten-Gehalt:15%) wurde bei Raumtemperatur unter Normaldruck mit einer Lösung von 0,6 mmol RuCl₂(=CHPh)(PCy₃)₂ in 20 ml CH₂Cl₂ umgesetzt. Hierbei wurde eine schwache Gasentwicklung beobachtet. Nach 1 h Rühren wurde die Lösung über Al₂O₃ chromatographiert und das farblose Filtrat destillativ von nicht umgesetzten Leichsiedern befreit. Man erhielt 96 g einer farblosen, niederviskosen Flüssigkeit folgender Zusammensetzung (GC-Flächenprozent):
4% C₇H₁₂, 11% C₈H₁₆, 14% C₁₀H₁₈, 3% C₁₂H₂₀, 8% C₁₃H₂₄, 12% C₁₅H₂₆, 2% C₁₇H₂₈, 5% C₁₈H₃₂, 9% C₂₀H₃₄, 1% C₂₂H₃₆, 4% C₂₃H₄₀, 7% C₂₅H₄₂, 3% C₂₈H₄₈, 6% C₃₀H₅₀, 1% C₃₃H₅₆, 4% C₃₅H₅₈, 3% C₄₀H₅₈, 3% C₄₀H₆₆, 2% C₄₀H₆₆, 1% C₄₀H₆₆.
Jodzahl: 329 g J₂/100 g

### Beispiel 3: (Modellsystem)

Ein 1:1-Gemisch aus Cyclopenten und 1-Penten wurde bei 60°C, 5 bar und Verweilzeiten von 1-3 h kontinuierlich in einen mit ReO₇/Al₂O₃ bestückten Rohrreaktor gepumpt. Mit Hilfe eines bei 115°C und Normaldruck betriebenen Fallfilmverdampfers wurde das Reaktionsprodukt anschließend in eine Leicht- und eine Hochsiederfraktion getrennt und erstere in den Metatheseprozeß zurückgeführt. Die Hochsiederfraktion wurde im Vakuum von Restmengen an Leichtsiedern befreit. Man erhielt bei Raum-Zeit-Ausbeuten von 50-500 g l⁻¹ h⁻¹ leicht gelbliche Flüssigkeiten, die abschließend über Al₂O₃ chromatographiert wurden. Eine entnommene Probe hatte folgende Zusammensetzung (GC-Flächenprozent):
3% C₇H₁₂, 9% C₈H₁₆, 16% C₁₀H₁₈, 2% C₁₂H₂₀, 8% C₁₃H₂₄, 13% C₁₅H₂₆, 2% C₁₇H₂₈, 6% C₁₈H₃₂, 11% C₂₀H₃₄, 1% C₂₂H₃₆, 4% C₂₃H₄₀, 9% C₂₅H₄₂, 2% C₂₈H₄₈, 6% C₃₀H₅₀, 3% C₃₅H₅₈, 2% C₄₀H₆₆, 1% C₄₀H₆₆, 1% C₄₅H₇₄. Jodzahl: 349 g J₂/100 g

### Beispiel 4: (heterogen katalysierte Metathese von C₅-Schnitt)

1 l C₅-Schnitt wurde bei 60°C, 5 bar und Verweilzeiten von 1-3 h kontinuierlich in einen mit Re₂O₇/Al₂O₃ bestückten Rohrreaktor gepumpt. Mit Hilfe eines bei 115°C und Normaldruck betriebenen Fallfilmverdampfers wurde das Reaktionsprodukt in eine Leicht- und eine Hochsiederfraktion getrennt. Letztere wurde durch Destillation im Vakuum von Restmengen an Leichtsiedern befreit. Man erhielt bei Raum-Zeit-Ausbeuten von 20-100 g l⁻¹ h⁻¹ und Cyclpenten-Umsätzen bis 70% leicht gelbliche Flüssigkeiten, die abschließend über Al₂O₃ chromatographiert wurden. Eine entnommene Probe hatte folgende Zusammensetzung (GC-Flächenprozent):
4% C₇H₁₂, 11% C₈H₁₆, 14% C₁₀H₁₈, 3% C₁₂H₂₀, 8% C₁₃H₂₄, 12% C₁₅H₂₆, 2% C₁₇H₂₈, 5% C₁₈H₃₂, 9% C₂₀H₃₄, 1% C₂₂H₃₆, 4% C₂₃H₄₀, 7% C₂₅H₄₂, 3% C₂₈H₄₈, 6% C₃₀H₅₀, 1% C₃₃H₅₆, 4% C₃₅H₅₈, 3% C₄₀H₆₆, 2% C₄₅H₇₄, 1% C₅₀H₈₂.
Jodzahl: 325 g J₂/100 g

### Beispiel 5:

1 l C₅-Schnitt wurde in äquimolarem Verhältnis mit Raffinat II bei 60°C, 11 bar und Verweilzeiten von 1-2 h kontinuierlich in einen mit Re₂O₇/Al₂O₃ bestückten Rohrreaktor gepumpt. Mit Hilfe eines bei 100°C und Normaldruck betriebenen Fallfilmverdampfers wurde das Reaktionsprodukt in eine Leicht- und eine Hochsiederfraktion getrennt. Letztere wurde durch Destillation im Vakuum von Restmengen an Leichtsiedern befreit. Man erhielt bei Raum-Zeit-Ausbeuten von 50-200 g l⁻¹ h⁻¹ und Cyclopenten-Umsätzen um 80% leicht gelbliche Flüssigkeiten, die abschließend über Al₂O₃ chromatographiert wurden. Die Proben wurden GC-analytisch untersucht und enthielten folgende, nicht mehr eindeutig zuzuordnende Produkt-Stammbäume:
C₆H₁₂, C₇H₁₂, C₈H₁₄, C₉H₁₆ + jeweilige Folgeprodukte bis C₄₉H₈₀. Jodzahl: 356 g J₂/100 g

### Beispiel 6: (kontinuierliche Ethenolyse von Cyclopenten)

Cyclopenten wurden unter 30 bar Ethen bei 60°C und Verweilzeiten von 1-2 h kontinuierlich in einen mit Re₂O₇/Al₂O₃ bestückten Rohrreaktor gepumpt. Mit Hilfe eines bei 80°C und Normaldruck betriebenen Fallfilmverdampfers wurde das Reaktionsprodukt in eine Leicht- und eine Hochsiederfraktion getrennt. Letztere wurde durch Destillation im leichten Vakuum von Restmengen an Leichsiedern befreit. Bei Raum-Zeit-Ausbeuten von 100-350 g l⁻¹h⁻¹ und Cyclopenten-Umsätzen um 85% wurden orangegelbe Flüssigkeiten erhalten, welche nach Chromatographie über Al₂O₃ folgende Zusammensetzung aufwiesen (GC-Flächenprozent):
29% C₇H₁₂, 4% C₈H₁₄, 24% C₁₂H₂O, 3% C₁₃H₂₂, 17% C₁₇H₂₈, 1% C₁₈H₃₀, 12% C₂₂H₃₆, 1% C₂₃H₃₈, 8% C₂₇H₄₄, 1% höhere Olefine.
Jodzahl: 384 g J₂/100 g

### Beispiel 7: (heterogen katalysierte, diskontinuierliche Ethenolyse von C₅-Schnitt)

In einem 100 ml Druckbehälter, welcher mit 10 g Re₂O₇/Al₂O₃ bestückt ist, wurden 60 ml C₅-Schnitt bei Raumtemperatur mit 30 bar Ethen behandelt. Nach Erwärmen auf 60°C wurde mit Ethen bis zu einem Gesamtdruck von 70 bar nachgepreßt und dieser im weiteren Reaktionsverlauf konstant gehalten. Nach 2 h wurde das Reaktionsgemisch auf Raumtemperatur abgekühlt und vorsichtig entspannt. Die so erhaltene farblose Lösung wies nach destillativer Abtrennung der Leichtsieder folgende Zusammensetzung auf (GC-Flächenprozent):
16% C₇H₁₂, 12% C₈H₁₄, 6% C₉H₁₆, 2% C₁₀H₁₈, 14% C₁₂H₂₀, 9% C₁₃H₂₂, 3% C₁₄H₂₄, 1% C₁₅H₂₆, 9% C₁₇H₂₈, 6% C₁₈H₃₀, 2% C₁₉H₃₂, 6% C₂₂H₃₆, 2% C₂₃H₃₈, 4% C₂₇H₄₄, 8% höhere Olefine.
Ausbeute: 11 g Oligomere
Jodzahl: 376 g J₂/100 g

### Beispiel 8: (homogen katalysierte, diskontinuierliche Ethenolyse von C₅-Schnitt)

In einem 100 ml Druckbehälter wurden 60 ml C₅-Schnitt bei Raumtemperatur mit einer Lösung von 84 mg (0,10 mmol) RuCl₂(=CHPh)(PCy₃)₂ in 2 ml CH₂Cl₂ versetzt und sofort 70 bar Ethen aufgepreßt. Durch regelmäßiges Nachpressen wurde der Druck im weiteren Reaktionsverlauf konstant gehalten. Nach 1 h Rühren bei Raumtemperatur wurde das Reaktionsgemisch vorsichtig entspannt. Die so erhaltene farblose Lösung wies nach destillativer Abtrennung der Leichtsieder folgende Zusammensetzung auf (GC-Flächenprozent):
13% C₇H₁₂, 12% C₈H₁₄, 6% C₉H₁₆, 1% C₁₀H₁₈, 10% C₁₂H₂₀, 11% C₁₃H₂₂, 4% C₁₄H₂₄, 9% C₁₇H₂₈, 8% C₁₈H₃₀, 3% C₁₉H₃₂, 4% C₂₃H₃₈, 3% C₂₇H₄₄, 10% höhere Olefine.
Ausbeute: 12 g Oligomere
Jodzahl 372 g J₂/100 g

## Patentansprüche

1. Verfahren zur Herstellung von Oligomerengemischen der Formel I 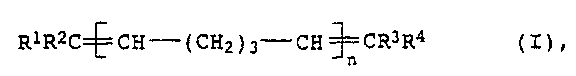 die von Cyclopenten abgeleitet sind,
worin
n für eine ganze Zahl von 1 bis 15 steht,
R¹, R², R³, R⁴ unabhängig voneinander für Wasserstoff oder Alkyl stehen,
**dadurch gekennzeichnet, daß** man ein Cyclopenten sowie acyclische Monoolefine enthaltendes, aus der Erdölverarbeitung durch Cracken erhaltenes Kohlenwasserstoffgemisch (C₅-Schnitt) mit einem Cyclopentengehalt von mindestens 5 Gew.-% und einem Anteil von Pentenisomeren an den acyclischen Monoolefinen von mindestens 70 Gew.-% in einer homogen oder heterogen katalysierten Metathesereaktion umsetzt.

2. Verfahren nach Anspruch 1, **dadurch gekennzeichnet, daß** die Metathesereaktion
a) die Disproportionierung der acyclischen Monoolefine des Kohlenwasserstoffgemisches durch Kreuzmetathese,
b) die Oligomerisation des Cyclopentens durch ringöffnende Metathese,
c) den Kettenabbruch durch Umsetzung der Oligomere aus b) mit einem acyclischen Olefin des Kohlenwasserstoffgemisches oder eines Produktes aus a),
umfaßt, wobei die Schritte a) und/oder b) und/oder c) mehrmals für sich alleine oder in Kombination durchlaufen werden können.

3. Verfahren nach einem der Ansprüche 1 oder 2, **dadurch gekennzeichnet, daß** man ein Kohlenwasserstoffgemisch mit einem Gesamtolefinanteil von mindestens 30 Gew.-% verwendet.

4. Verfahren nach Anspruch 3, **dadurch gekennzeichnet, daß** man ein Kohlenwasserstoffgemisch mit einem Cyclopentengehalt von mindestens 10 Gew.-% verwendet.

5. Verfahren nach einem der Ansprüche 3 oder 4, **dadurch gekennzeichnet, daß** man ein Kohlenwasserstoffgemisch verwendet, wobei der Anteil von Pentenisomeren an den acyclischen Monoolefinen mindestens 80 Gew.-% beträgt.

6. Verfahren nach einem der Ansprüche 1 oder 2, **dadurch gekennzeichnet, daß** man ein Kohlenwasserstoffgemisch verwendet, welches den C₅-Schnitt und eine acyclische C₄-Olefine enthaltende Erdölfraktion (Raffinat-2) umfaßt.

7. Verfahren nach einem der Ansprüche 1 oder 2, **dadurch gekennzeichnet, daß** man ein Kohlenwasserstoffgemisch verwendet, welches den C₅-Schnitt und Ethen umfaßt.

8. Verfahren nach einem der Ansprüche 1 bis 7, **dadurch gekennzeichnet, daß** man einen Katalysator verwendet, der mindestens ein Übergangsmetall der 6., 7. oder 8. Nebengruppe umfaßt.

9. Verfahren nach Anspruch 8, **dadurch gekennzeichnet, daß** man einen Katalysator verwendet, der
a) mindestens einen Übergangsmetallalkyliden-Komplex (Übergangsmetallcarbon-Komplex) oder
b) eine zur Bildung eines Komplexes a) geeignete Kombination aus einer Übergangsmetallverbindung und wenigstens einem weiteren Agens, bevorzugt einem Alkylierungsmittel, oder
c) eine zur Bildung eines Komplexes a) mit den im Kohlenwasserstoffgemisch enthaltenen Olefinen geeignete Übergangsmetallverbindung
umfaßt.

10. Verfahren nach einem der Ansprüche 8 oder 9, **dadurch gekennzeichnet, daß** man einen homogenen Katalysator, ausgewählt unter Rutheniumalkylidenkomplexen der Formel RuCl₂(=CHR)(PR'₃), Molybdänalkylidenkomplexen, sowie CH₃ReO₃/C₂H₅AlCl₂, WOCl₂(O-[2,6-Br₂-C₆H₃])/Sn(CH₃)₄, WCl₆/C₂H₅AlCl₂(Sn(CH₃)₄)/EtOH, WOCl₄/Sn(CH₃)₄ verwendet.

11. Verfahren nach einem der Ansprüche 8 oder 9, **dadurch gekennzeichnet, daß** man einen heterogenen Katalysator, ausgewählt unter Re₂O₇ und CH₃ReO₃ auf einem anorganischen Träger verwendet.

12. Verfahren nach Anspruch 7, **dadurch gekennzeichnet, daß** die Reaktion bei einem Druck von 1 bis 200 bar durchgeführt wird.

13. Verfahren nach Anspruch 7, **dadurch gekennzeichnet, dass** die Reaktion bei einem Druck von 20 bis 150 bar durchgeführt wird.

14. Verfahren nach Anspruch 7, **dadurch gekennzeichnet, dass** die Reaktion bei einem Druck von 40 bis 100 bar durchgeführt wird.

15. Cyclopenten-Oligomerengemische der Formel I, erhältlich durch ein Verfahren nach einem der Ansprüche 1 bis 14.

16. Verwendung der Cyclopenten-Oligomerengemische nach Anspruch 15 als Zwischenprodukte für die Weiterverarbeitung durch Funktionalisierung wenigstens eines Teils der enthaltenen Doppelbindungen.

## Claims

1. A process for preparing oligomer mixtures of the formula I 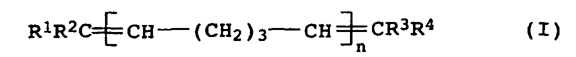 which are derived from cyclopentene,
where
n is an integer from 1 to 15,
R¹, R², R³, R⁴ independently of one another are hydrogen or alkyl,
which comprises reacting, in a homogeneously or heterogeneously catalyzed metathesis reaction, a hydrocarbon mixture which contains cyclopentene and acyclic monoolefins and originates from petroleum refining by cracking (C₅ fraction) and has a cyclopentene content of at least 5% by weight and a pentene isomer content of the acyclic monoolefins of at least 70% by weight.

2. A process as claimed in claim 1, wherein the metathesis reaction consists of
a) the disproportionation of the acyclic monoolefins of the hydrocarbon mixture by cross metathesis,
b) the oligomerization of the cyclopentene by ring-scission metathesis,
c) chain termination by reacting the oligomers from b) with an acyclic olefin of the hydrocarbon mixture or of a product from a),
where the steps a) and/or b) and/or c) can proceed repeatedly, alone or in combination.

3. A process as claimed in one of claims 1 or 2, wherein a hydrocarbon mixture having a total olefin content of at least 30% by weight is used.

4. A process as claimed in claim 3, wherein a hydrocarbon mixture having a cyclopentene content of at least 10% by weight is used.

5. A process as claimed in either claim 3 or 4, wherein the pentene isomer content of the acyclic monoolefins is at least 80% by weight.

6. A process as claimed in either claim 1 or 2, wherein a hydrocarbon mixture is used which comprises the C₅ fraction and a petroleum fraction containing acyclic C₄ olefins (raffinate 2).

7. A process as claimed in either claim 1 or 2, wherein a hydrocarbon mixture is used which comprises the C₅ fraction and ethene.

8. A process as claimed in one of claims 1 to 7, wherein a catalyst is used which comprises at least one transition metal of subgroups 6, 7 or 8.

9. A process as claimed in claim 8, wherein a catalyst is used which comprises
a) at least one transition metal-alkylidene complex (transition metal-carbon complex) or
b) a combination, suitable for forming a complex a), of a transition metal compound and at least one further agent, preferably an alkylating agent, or
c) a transition metal compound suitable for forming a complex a) with the olefins present in the hydrocarbon mixture.

10. A process as claimed in either claim 8 or 9, wherein a homogeneous catalyst is used which is selected from the group consisting of ruthenium-alkylidene complexes of the formula RuCl₂(=CHR)(PR'₃), molybdenum-alkylidene complexes, and CH₃ReO₃/C₂H₅AlCl₂, WOCl₂(O-[2,6-Br₂-C₆H₃])/Sn(CH₃)₄, WCl₆/C₂H₅AlCl₂(Sn(CH₃)₄)/EtOH, WOCl₄/Sn(CH₃)₄.

11. A process as claimed in either claim 8 or 9, wherein a heterogeneous catalyst is used which is selected from the group consisting of Re₂O₇ and CH₃ReO₃ on an inorganic support.

12. A process as claimed in claim 7, wherein the reaction is carried out at a pressure of from 1 to 200 bar.

13. A process as claimed in claim 7, wherein the reaction is carried out at a pressure of from 20 to 150 bar.

14. A process as claimed in claim 7, wherein the reaction is carried out at a pressure of from 40 to 100 bar.

15. A cyclopentene oligomer mixture of the formula I, obtainable by a process as claimed in one of claims 1 to 14.

16. The use of cyclopentene oligomer mixtures as claimed in claim 15 as intermediates for the further processing by functionalization of at least some of the double bonds present.

## Revendications

1. Procédé de préparation de mélanges d'oligomères de la formule I 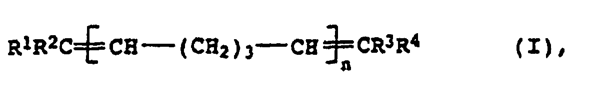 dérivés de cyclopentène,
où
n est un nombre entier de 1 à 15,
R¹, R², R³ et R⁴ représentent indépendamment les uns des autres de l'hydrogène ou un radical alkyle,
**caractérisé en ce que** l'on fait réagir un mélange d'hydrocarbures (fraction en C₅) contenant du cyclopentène et des monooléfines acycliques, obtenu par craquage lors du traitement du pétrole, d'une teneur en cyclopentène d'au moins 5% en poids et contenant une fraction d'isomères de pentène par rapport aux monooléfines acycliques d'au moins 70% en poids, par une réaction de métathèse à catalyse homogène ou hétérogène.

2. Procédé selon la revendication 1, **caractérisé en ce que** la réaction de métathèse comprend
a) la disproportionation des monooléfines acycliques du mélange d'hydrocarbures par métathèse croisée,
b) l'oligomérisation du cyclopentène par métathèse à décyclisation,
c) la rupture de chaîne par réaction des oligomères de b) avec une oléfine acyclique du mélange d'hydrocarbures ou un produit de a),
les étapes a) et/ou b) et/ou c) pouvant être entreprises plusieurs fois isolément ou en combinaison.

3. Procédé selon l'une des revendications 1 ou 2, **caractérisé en ce que** l'on utilise un mélange d'hydrocarbures contenant une fraction totale d'oléfines d'au moins 30% en poids.

4. Procédé selon la revendication 3, **caractérisé en ce que** l'on utilise un mélange d'hydrocarbures d'une teneur en cyclopentène d'au moins 10% en poids.

5. Procédé selon l'une des revendications 3 ou 4, **caractérisé en ce que** l'on utilise un mélange d'hydrocarbures dans lequel la fraction d'isomères du pentène par rapport aux monooléfines acycliques est d'au moins 80% en poids.

6. Procédé selon l'une des revendications 1 ou 2, **caractérisé en ce que** l'on utilise un mélange d'hydrocarbures comprenant la fraction en C₅ et une fraction de pétrole (produit raffiné 2) contenant des oléfines acycliques en C₄.

7. Procédé selon l'une des revendications 1 ou 2, **caractérisé en ce que** l'on utilise un mélange d'hydrocarbures comprenant la fraction en C₅ et de l'éthène.

8. Procédé selon l'une des revendications 1 à 7, **caractérisé en ce que** l'on utilise un catalyseur comprenant au moins un métal de transition des 6^{ème}, 7^{ème} ou 8^{ème} sous-groupes.

9. Procédé selon la revendication 8, **caractérisé en ce que** l'on utilise un catalyseur comprenant
a) au moins un complexe alkylidène de métal de transition (complexe carbone - métal de transition) ou
b) une combinaison appropriée à la formation d'un complexe a), constituée d'un composé de métal de transition et d'au moins un autre agent, de préférence un agent d'alkylation, ou
c) un composé de métal de transition approprié à la formation d'un complexe a) avec les oléfines contenues dans le mélange d'hydrocarbures.

10. Procédé selon l'une des revendications 8 ou 9, **caractérisé en ce que** l'on utilise un catalyseur homogène choisi parmi des complexes alkylidène - ruthénium de la formule RuCl₂(=CHR)(PR'₃), des complexes alkylidène - molybdène, ainsi que CH₃ReO₃/C₂H₅AlCl₂, WO Cl₂(O-[2,6-Br₂-C₆H₃])/Sn(CH₃)₄, WCl₆/C₂H₅AlCl₂(Sn(CH₃)₄/EtOH, WOCl₄/Sn(CH₃)₄.

11. Procédé selon l'une des revendications 8 ou 9, **caractérisé en ce que** l'on utilise un catalyseur hétérogène, choisi parmi Re₂O₇ et CH₃ReO₃ sur un support inorganique.

12. Procédé selon la revendication 7, **caractérisé en ce que** la réaction est entreprise sous une pression de 1 à 200 bar.

13. Procédé selon la revendication 7, **caractérisé en ce que** la réaction est entreprise sous une pression de 20 à 150 bar.

14. Procédé selon la revendication 7, **caractérisé en ce que** la réaction est entreprise sous une pression de 40 à 100 bar.

15. Mélanges d'oligomères de cyclopentène de la formule I, que l'on peut obtenir par un procédé selon l'une des revendications 1 à 14.

16. Utilisation des mélanges d'oligomères de cyclopentène selon la revendication 15 comme produits intermédiaires pour le retraitement par fonctionnalisation d'une partie des doubles liaisons obtenues.
